# EUROPEAN PATENT SPECIFICATION

(11) **EP 4 049 620 B1**
(45) Date of publication and mention of the grant of the patent: **04.02.2026**
(21) Application number: 20898858.4
(22) Date of filing: 10.12.2020
(51) Int. Cl.: A61F 2/08

(54) **SELF-SWITCHING TRANSMISSION ASSEMBLY, BALLOON, AND PROSTHESIS FOR USE IN SHOULDER JOINT**
SELBSTSCHALTENDE ÜBERTRAGUNGSANORDNUNG, BALLON UND PROTHESE ZUR VERWENDUNG IM SCHULTERGELENK
ENSEMBLE DE TRANSMISSION À COMMUTATION AUTOMATIQUE, BALLONNET ET PROTHÈSE DESTINÉS À ÊTRE UTILISÉS DANS UNE ARTICULATION D'ÉPAULE

(30) Priority: 11.12.2019 CN 201911268570
(43) Date of publication of application: 31.08.2022
(73) Proprietor: Shanghai Endophix Co., Ltd., Shanghai 201203 (CN)
(72) Inventor: JI, Xiaofei, Shanghai 201203 (CN); LIU, Chen, Shanghai 201203 (CN); YUE, Bin, Shanghai 201203 (CN)
(74) Representative: Marks & Clerk LLP
(86) International application number: PCT/CN2020/135270
(87) International publication number: WO 2021/115378

(56) References cited:
- CN-A- 1 547 650
- CN-A- 104 136 820
- CN-A- 105 722 765
- CN-A- 105 722 765
- CN-A- 107 035 875
- CN-A- 109 758 269
- CN-A- 109 758 269
- CN-U- 204 025 702
- CN-U- 212 089 839
- US-A- 4 466 550
- US-A- 5 950 986
- US-A1- 2006 149 380

## Description

### TECHNICAL FIELD

The present application relates to the field of medical devices, in particular to a medical self-on-off transmission assembly, a balloon and a prosthesis for a shoulder joint.

### BACKGROUND

With the aging of the population and the increase in the number of exercising people, the incidence of rotator cuff tear is increasing year by year. At the same time, elderly patients often rarely receive treatment intervention in the early stage due to various reasons such as concept and economic level. Once the disease progresses to the point where surgery is required, it has often developed into a large or irreparable rotator cuff tear, and the treatment will cost more. This not only brings physical pain and economic burden to patients and affects the quality of life of patients, but also gradually becomes a major social health problem.

The rotator cuff supports and stabilizes the scapulohumeral joint during the movement of the shoulder joint. Rotator cuff injury may weaken or even lose the fulcrum function for maintaining the humeral head and the glenoid, which may seriously affect the function of the shoulder joint. When the rotator cuff injury occurs, implantation of a rotator cuff balloon at the joint can be considered. The rotator cuff balloon is mainly used to be implanted between the acromion and the humeral head to ensure a smooth joint movement, reduce friction, and restore shoulder biomechanics.

During the implantation operation, the rotator cuff balloon needs to be folded in a puncture cannula. After being positioned and released under a direct vision of an arthroscope, the balloon is filled by injecting physiological saline through a catheter. After separating the catheter from the balloon, the catheter is withdrawn and the balloon is sealed. Therefore, the design of the rotator cuff balloon needs to ensure that the balloon is disconnected from the catheter without leakage, after being filled. However, it is not easy to separate the rotator cuff balloon, that is currently used clinically, from the catheter, or the filling fluid inside the balloon is easy to flow out from a connection between the rotator cuff balloon and the catheter after the balloon is filled.
US2006/149380A1 relates to a stent for facilitating regeneration of an intervertebral nucleus.

### SUMMARY

Accordingly, it is necessary to provide a self-on-off transmission assembly, a balloon and a prosthesis for a shoulder joint, to address problems that the rotator cuff balloon is not easily separated from the catheter and the sealing performance of the rotator cuff balloon is poor.

In an aspect, a medical self-on-off transmission assembly is provided, and includes: a communication tube, including an input end and an output end; an outer tube, an end of the outer tube being configured to be capable of being inserted into the communication tube from the input end and being moved out of the communication tube from the input end; and a sealing tube configured to be capable of being placed into a tube body of the communication tube and being moved in an axial direction with respect to the tube body of the communication tube, the sealing tube including a connecting end and a sealing end. The connecting end is configured to be detachably connected to the end of the outer tube through a connecting-driving mechanism. The sealing end is provided with a communicating area. When the communicating area is exposed to the output end, the self-on-off transmission assembly is in an on-state; when the communicating area is placed in the tube body of the communication tube, the self-on-off transmission assembly is in a sealed off-state. The outer tube is configured to at least allow the self-on-off transmission assembly to be switchable from being in the on-state to being in the sealed off-state, by driving the sealing tube to move along the communication tube by the connecting-driving mechanism.

Further, the connecting-driving mechanism includes a first member arranged at the end of the outer tube, and a second member arranged at the connecting end of the sealing tube, and the first member and the second member are configured to be capable of being coupled with each other in a circumferential direction and decoupled from each other in the axial direction.

Further, the connecting-driving mechanism includes a third member arranged at the end of the outer tube, and a fourth member arranged at the connecting end of the sealing tube, and the third member and the fourth member are configured to be capable of being coupled with each other in the axial direction and in a circumferential direction, and being decoupled from each other in the axial direction and in the circumferential direction.

Further, the connecting-driving mechanism includes a plug-in cannula arranged at the connecting end of the sealing tube. The plug-in cannula is configured to be capable of being inserted into an inner hole of the end of the outer tube. When the end of the outer tube is inserted into the communication tube, the end of the outer tube is clamped between the communication tube and the plug-in cannula.

Further, the first member includes a first arc-shaped cylinder, the second member includes a second arc-shaped cylinder, and the first arc-shaped cylinder and the second arc-shaped cylinder are capable of being abutted against each other in the circumferential direction.

Further, an outer peripheral wall of the end of the outer tube and an outer peripheral wall of the sealing tube are both provided with outer threads. An inner hole wall of the communication tube is provided with inner threads matched with the outer threads.

Further, the third member is a first buckle, the fourth member is a second buckle, and the first buckle and the second buckle are capable of being engaged with each other.

Further, the first buckle and the second buckle are hooks extending in the circumferential direction.

Further, an inner hole of the communication tube includes a cylindrical hole and a tapered hole which are connected to each other. The tapered hole is close to the output end. The sealing tube includes a cylindrical tube body and an inverted tapered tube body which are connected to each other. The fourth member is arranged on the cylindrical tube body. The communicating area is arranged in the inverted tapered tube body. The cylindrical tube body is slidably matched with the cylindrical hole. The inverted tapered tube body is adapted to be abutted against the tapered hole.

Further, the sealing tube includes a cylindrical tube body and an inverted tapered tube body which are connected to each other. The fourth member and the communicating area are arranged in the cylindrical tube body. The cylindrical tube body is slidably matched with an inner hole of the communication tube. The inverted tapered tube body is adapted to be abutted against an end of the inner hole of the communication tube.

Further, the sealing tube includes: a sealing ring connected to the plug-in cannula, an outer periphery surface of the sealing ring being sealingly fitted with an inner hole wall of the communication tube; an end ring, the communicating area being arranged on an periphery wall of the end ring, an outer periphery surface of the end ring being capable of being sealingly fitted with the inner hole wall of the communication tube; and a transition ring connected between the sealing ring and the end ring, a diameter of an outer periphery of the transition ring being smaller than a diameter of an inner hole of the communication tube.

Further, a first sealing structure is arranged between an outer peripheral surface of the outer tube and an inner hole wall of the communication tube, and a second sealing structure is arranged between an outer peripheral surface of the sealing tube and the inner hole wall of the communication tube.

Further, the first sealing structure is at least one of a threaded fitting seal, an interference fit seal, and an elastic fit seal.

Further, the second sealing structure includes outer threads provided on the outer peripheral surface of the sealing tube, and inner threads provided on the inner hole wall of the communication tube and matched with the outer threads. Alternatively, the second sealing structure is an inverted tapered tube body arranged on the sealing tube close to the sealing end, a maximum circumferential dimension of the inverted tapered tube body is greater than a circumferential dimension of an inner hole of the communication tube. Alternatively, the second sealing structure includes a sealing ring being sleeved on the sealing tube. An outer peripheral surface of the sealing ring is sealingly fitted with the inner hole wall of the communication tube.

Further, the communicating area is a through hole or a slit located in a periphery wall of the sealing tube, and the communicating area is capable of being blocked by an inner hole wall of the communication tube.

Further, a port of the sealing end of the sealing tube is sealed by a sealing plate.

In another aspect, a balloon is provided, and includes the medical self-on-off transmission assembly as described above, and a balloon body. The output end of the communication tube is connected to an opening of the balloon body.

Further, the communication tube, the outer tube, the sealing tube and the balloon body are made of polyamide, polyester, polyurethane, polyamide polyether block copolymer, polyethylene, polypropylene, polyimide, cross-linked polyethylene, cross-linked polyurethane, ionomer, polycaprolactone (PCL), polylactic acid (PLA), polylactic acid-glycolic acid copolymer (PLGA), lactide-caprolactone copolymer (PLCL), plastic starch materials, polyionic complexes, or copolymers or blends of multiple of the above materials.

In another aspect, a prosthesis for a shoulder joint is provided, and includes a balloon, a catheter, and a catheter connector that connects the balloon to the catheter. The catheter connector includes: a communication tube including an input end and an output end; an outer tube, an end of the outer tube being configured to be capable of being inserted into the communication tube from the input end and being moved out of the communication tube from the input end; and a sealing tube, configured to be capable of being placed into a tube body of the communication tube and being moved in an axial direction with respect to the tube body of the communication tube. The sealing tube includes a connecting end and a sealing end. The connecting end is configured to be detachably connected to the end of the outer tube through a connecting-driving mechanism. The sealing end is provided with a communicating area. When the communicating area is exposed to the output end, the catheter connector is in an on-state; when the communicating area is placed in the tube body of the communication tube, the catheter connector is in a sealed off-state. The outer tube is configured to at least allow the catheter connector to be switchable from being in the on-state to being in the sealed off-state, by driving the sealing tube to move along the communication tube by the connecting-driving mechanism.

### BRIEF DESCRIPTION OF THE DRAWINGS

FIG. 1 is a perspective exploded view of a self-on-off transmission assembly according to a first embodiment of the present application.
FIG. 2 is a schematic view of the self-on-off transmission assembly of FIG. 1 combining with a balloon body.
FIG. 3 is a perspective exploded view of a self-on-off transmission assembly according to a second embodiment of the present application.
FIG. 4 is a cross-sectional view illustrating that an outer tube, a sealing tube and a communication tube according to the second embodiment of the present application are fitted and assembled.
FIG. 5 is a schematic view of the self-on-off transmission assembly of FIG. 3 combining with a balloon body.
FIG. 6 is a perspective exploded view of a self-on-off transmission assembly according to a third embodiment of the present application.
FIG. 7 is a schematic view of the self-on-off transmission assembly of FIG. 6 combining with a balloon body.

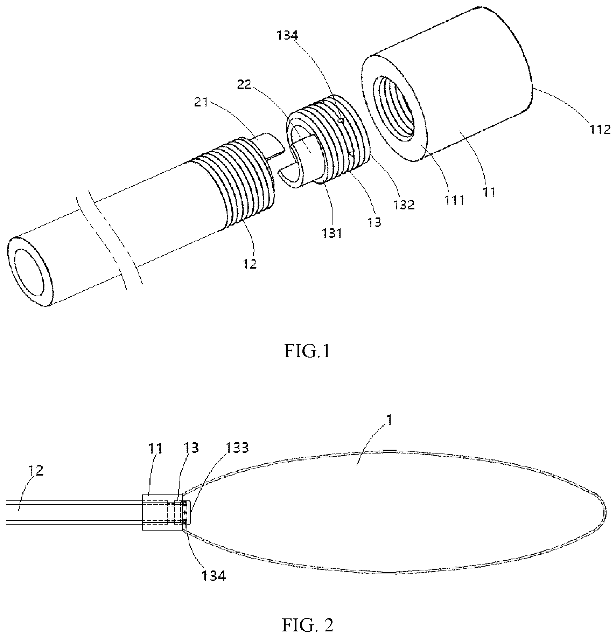

In FIGS. 1 to 2, 1- balloon body, 11- communication tube, 111- input end, 112-output end, 12- outer tube, 13- sealing tube, 131- connecting end, 132- sealing end, 133-sealing plate, 134- through hole, 21- first arc-shaped cylinder, 22- second arc-shaped cylinder,

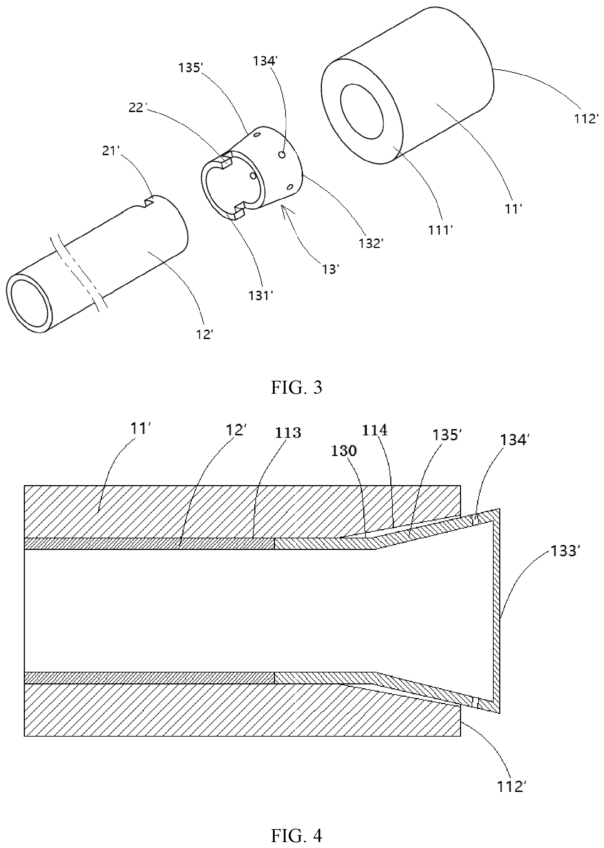

In FIGS. 3 to 5, 1'- balloon body, 11'- communication tube, 111'- input end, 112'- output end, 113- cylindrical hole, 114- tapered hole, 12'- outer tube, 13'- sealing tube, 130- cylindrical tube body, 131'- connecting end, 132'- sealing end, 133'- sealing plate, 134'- through hole, 135'- inverted tapered tube body, 21'- first buckle, 22'- second buckle,

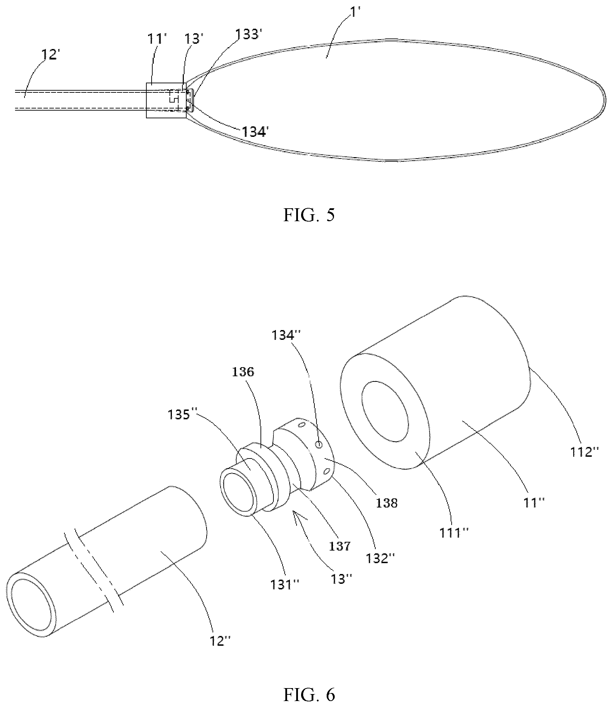

In FIGS. 6 to 7, 1"- balloon body, 11"- communication tube, 111"- input end, 112"- output end, 12"- outer tube, 13"- sealing tube, 131"- connecting end, 132"- sealing end, 133"- sealing plate, 134"- through hole, 135"- plug-in cannula, 136- sealing ring, 137-transition ring, 138- end ring.

### DETAILED DESCRIPTION OF THE EMBODIMENTS

The present application will be further described below with reference to the accompanying drawings.

In order to facilitate understanding of the present application, the present application will be described more fully below with reference to the accompanying drawings. Preferred embodiments of the present application are shown in the accompanying drawings. However, the present application may be implemented in many different forms and is not limited to the embodiments described herein. Those of ordinary skill in the art will appreciate that changes and modifications can be made to the various embodiments described herein without departing from the scope of the present application, defined by the appended claims. In addition, descriptions of well-known functions and constructions may be omitted for clarity and conciseness.

It will be understood that when an element is referred to as being "fixed to" another element, it can be directly on another element or an intermediate element may also be present. When an element is referred to as being "connected to" another element, the element can be directly connected to another element or an intermediate element may be present at the same time.

Unless otherwise defined, all technical and scientific terms used herein have the same meaning as commonly understood by one of ordinary skill in the technical field to which this application belongs. The terms used herein in the specification of this application are for the purpose of describing specific embodiments only, and are not intended to limit this application. As used herein, the term "and/or" includes any and all combinations of one or more of the associated listed items.

A balloon according to the present application, such as a rotator cuff balloon, may be used as a prosthesis for reducing human soft tissue damage; and a balloon according to the present application, such as a gastric balloon, may be used as a prosthesis for filling human cavities or capsular bags.

As shown in FIG. 1, a first embodiment of the present application provides a self-on-off transmission assembly, which includes a communication tube 11, an outer tube 12 and a sealing tube 13. The communication tube 11 is provided with a space therein, and the outer tube 12 and the sealing tube 13 may pass through and be received in the space. The outer tube 12 is used for delivering the filling material. The communication tube 11 includes an input end 111 and an output end 112. An end of the outer tube 12 may be inserted into and moved out of the communication tube 11 from the input end 111. A first sealing structure capable of enhancing the sealing effect is arranged between an outer peripheral surface of the outer tube 12 and an inner hole wall of the communication tube 11. In the illustrated embodiment, the first sealing structure is a threaded fitting seal. For example, the outer peripheral surface of the outer tube 12 and the inner hole wall of the communication tube 11 are respectively provided with mutually matched outer threads and inner threads, so that a sealed connection between the outer tube 12 and the communication tube 11 may be realized by screwing the outer tube 12 into the communication tube 11. The sealing tube 13 is configured to be placeable into a tube body of the communication tube 11 and to be moveable in an axial direction with respect to the tube body of the communication tube. The sealing tube 13 includes a connecting end 131 and a sealing end 132. The connecting end 131 may be detachably connected to the end of the outer tube 12 through a connecting-driving mechanism. The outer peripheral surface of the sealing tube 13 and the inner hole wall of the communication tube 11 are sealingly fitted with each other by a second sealing structure. For example, the mutually matched outer threads and inner threads are respectively provided on the outer peripheral surface of the sealing tube 13 and the inner hole wall of the communication tube 11, so that the sealing tube 13 and the communication tube 11 may be sealingly connected by the thread connection. A port of the sealing end 132 is provided with a sealing plate 133, which closes the port.

The sealing end 132 is provided with a communicating area. In this embodiment, the communicating area is specifically a through hole 134 provided on a circumferential surface (peripheral wall) of the sealing tube 13 and communicating with an inner lumen and an outer wall of the sealing tube 13. It can be understood that, in other embodiments, the communicating area may be a slit in the circumferential surface of the sealing tube 13, which is not limited herein.

The through hole 134 is exposed to the output end 112 when the self-on-off transmission assembly is in an on-state, the filling material in the sealing tube 13 may flow out through the through hole 134, and the through hole 134 is in an on-state. In addition, the outer tube 12 may drive the sealing tube 13 to move from the output end 112 of the communication tube 11 to the input end 111 of the communication tube 11 by the connecting-driving mechanism until the through hole 134 is placed in the tube body of the communication tube 11, so that the through hole is switched from being in the on-state to being in a sealed off-state, while the self-on-off transmission assembly is in the sealed off-state.

When the self-on-off transmission assembly is in the on-state, the through hole 134 is exposed to the output end 112. In this case, an end of the outer tube 12 away from the sealing tube 13 may be communicated to an injection device. The filling material output from the injection device, such as liquid or gas, may be transmitted to the sealing tube 13 through the outer tube 12, and then flow out of the output end 112 of the communication tube 11 through the through holes 134 in the circumferential surface of the sealing tube 13. Since the first sealing structure and the second sealing structure have a sealing effect, the risk of leakage of the filling material between the outer tube 12, the sealing tube 13 and the communication tube 11 is reduced. When the on-state is not required, the outer tube 12 may drive the sealing tube 13 to move from the output end 112 of the communication tube 11 to the input end 111 of the communication tube 11 through the connecting-driving mechanism. When the outer tube 12 is moved to disconnect the threaded connection between the outer tube 12 and the communication tube 11, the connection with driving effect between the outer tube 12 and the sealing tube 13 can be easily released. In this case, the through hole 134 of the sealing tube 13 is placed in the tube body of the communication tube 11 and thus is sealed by the inner hole wall of the communication tube 11, and the output end 112 may be sealed by the sealing plate 133, so that the filling material located at the input end 111 of the communication tube 11 may not flow out from the output end 112 of the communication tube 11, and the external material may not enter the communication tube 11 through the output end 112, and the self-on-off transmission assembly is in the sealed off-state.

In the above-mentioned embodiment, the connecting-driving mechanism is a circumferential coupling structure. The circumferential coupling structure includes a first member arranged at the end of the outer tube 12 facing the sealing tube 13, and a second member arranged at the connecting end 131 of the sealing tube 13. The first member and the second member are configured to be coupled with each other in a circumferential direction and decoupled from each other in the axial direction. For example, the first member is a first arc-shaped cylinder 21, the second member is a second arc-shaped cylinder 22, and the first arc-shaped cylinder 21 and the second arc-shaped cylinder 22 may abut against each other in the circumferential direction. In some embodiments, the first arc-shaped cylinder 21 and the second arc-shaped cylinder 22 may be spliced into a complete cylinder in the circumferential direction. In other embodiments, the first arc-shaped cylinder 21 and the second arc-shaped cylinder 22 may be spliced into an incomplete cylinder in the circumferential direction. When the outer tube 12 is rotated, the sealing tube 13 may be driven to rotate synchronously through the cooperation of the first arc-shaped cylinder 21 on the outer tube 12 with the second arc-shaped cylinder 22 on the sealing tube 13. While being rotated, the outer tube 12 and the sealing tube 13 may be moved from the output end 112 of the communication tube 11 to the input end 111 of the communication tube 11 through a threaded cooperation with the inner hole wall of the communication tube 11. When the outer tube 12 is moved to be completely exposed to the input end 111 of the communication tube 11, the outer tube 12 may be disconnected from the sealing tube 13 by being pulled in the axial direction. Certainly, those of ordinary skill in the art can easily replace the shapes of the first arc-shaped cylinder 21 and the second arc-shaped cylinder 22 with other shapes, for example, the first member is a protrusion, and the second member has a concave shape matched with the protrusion, as long as the first member and the second member may be coupled with each other in the circumferential direction and decoupled from each other in the axial direction.

In the first embodiment, six through holes 134 are provided, for example. The plurality of through holes 134 are convenient to improve the speed of material transmission. In some other embodiments, the number of the through holes 134 may be set as needed, which is not limited herein.

The balloon shown in FIG. 2 may be obtained by arranging the self-on-off transmission assembly on the balloon body 1. In FIG. 2, the output end 112 of the communication tube 11 is connected to an opening of the balloon body 1. The outer tube 12 is pre-inserted into the communication tube 11 and kept coupled with the sealing tube 13 in the circumferential direction. The balloon body 1 is folded in a puncture cannula, delivered to a preset part of the human body and released under direct vision of an endoscope (e.g., an arthroscope). In this case, the self-on-off transmission assembly is in the on-state, and the balloon body 1 may be filled by injecting physiological saline through the outer tube 12, and then the outer tube 12 is rotated, and the outer tube 12 may drive the sealing tube 13 to rotate synchronously and to move from the output end 112 of the communication tube 11 to the input end 111 of the communication tube 11. When the outer tube 12 is moved to be completely exposed to the input end 111 of the communication tube 11, the outer tube 12 may be pulled in the axial direction to disconnect the outer tube 12 from the sealing tube 13. In this case, the through hole 134 is placed in the tube body of the communication tube 11 and thus is sealed by the inner hole wall of the communication tube 11, and the self-on-off transmission assembly is in the sealed off-state, the input end 111 of the communication tube 11 and the output end 112 of the communication tube 11 are not in communication with each other, and the physiological saline in the balloon body 1 may not flow out of the opening.

According to the balloon of the first embodiment, the separation and withdrawal of the outer tube 12 and the sealing of the balloon body 1 may be performed simultaneously. While the outer tube 12 is disconnected from the sealing tube 13, the through holes 134 of the sealing tube 13 are moved into the tube body of the communication tube 11 so as to be blocked, so that the self-on-off transmission assembly is in the sealed off-state. The opening of the balloon body 1 may be directly sealed by the sealing plate 133, and the sealing effect is good.

### Second Embodiment

A second embodiment shown in FIG. 3 to FIG. 5 provides another self-on-off transmission assembly and a balloon, the main structure of which is generally similar to those described in the first embodiment, and the main difference from those described in the first embodiment lies in structural forms of the first sealing structure, the second sealing structure and the connecting-driving mechanism. The first sealing structure between the outer peripheral surface of the outer tube 12' and the inner hole wall of the communication tube 11' is an interference fit seal. Specifically, the outer tube 12' is inserted into the communication tube 11' from the input end 111', and the outer peripheral surface of the outer tube 12' is in interference fit with the inner hole wall of the communication tube 11' to achieve sealing.

In addition, in the second embodiment, the second sealing structure between the outer peripheral surface of the sealing tube 13' and the inner hole wall of the communication tube 11' is an inverted tapered tube body 135' arranged on the sealing tube 13' close to the sealing end 132'. The inverted tapered tube body 135' may be in an interference fit with the inner hole wall of the communication tube 11'. Referring to FIG. 4, the sealing tube 13' includes a cylindrical tube body 130 connected with the inverted tapered tube body 135'. The cylindrical tube body 130 is used to be detachably connected to an end of the outer tube 12' through the connecting-driving mechanism. The through hole 134' is provided on the peripheral wall of the inverted tapered tube body 135'. The circumferential dimension of the inverted tapered tube body 135' gradually increases in an axial direction away from the cylindrical tube body 130. An inner hole of the communication tube 11' includes a cylindrical hole 113. The cylindrical tube body 130 of the sealing tube 13' is slidably matched with the cylindrical hole 113. When the outer tube 12' drives the sealing tube 13' to move from an output end 112' of the communication tube 11' to an input end 111' of the communication tube 11' along the cylindrical hole 113 through the connecting-driving mechanism, the outer peripheral surface of the inverted tapered tube body 135' may be abutted on the inner hole wall of the communication tube 11', so as to realize the sealed off-state of the through hole 134'. In particular, when the self-on-off transmission assembly is applied into the balloon, the balloon body 1' implanted into the predetermined part is subjected to a pressure, and then the filling liquid in the balloon body 1' exerts a pressure on the sealing plate 133', so that the connection between the inverted tapered tube body 135' and the communication tube 11' is tighter. The interference fit has good stability and good sealing effect. Preferably, the sealing tube 13' is made of polyamide and is integrally formed. The communication tube 11' is made of polyethylene material. The sealing tube 13' and the communication tube 11' are flexible and deformable, so that it is easier to operate the outer tube 12' to drive the sealing tube 13' to move from the output end 112' of the communication tube 11' to the input end 111' of the communication tube 11', and the sealing performance due to the close contact between the outer tube 12' and the communication tube 11' is better.

In this embodiment, the inner hole of the communication tube 11' has a tapered hole 114 matched with the inverted tapered tube body 135'. The tapered hole 114 is connected to the cylindrical hole 113, and the tapered hole 114 is close to the output end 112'. When the outer tube 12' drives the sealing tube 13' to move from the output end 112' of the communication tube 11' to the input end 111' of the communication tube 11' through the connecting-driving mechanism, the outer peripheral surface of the inverted tapered tube body 135' may be just fitted and embedded into the tapered hole 114 of the communication tube 11', so as to achieve the sealed off-state of the through hole 134'. However, in other embodiments, the through hole 134' may be provided on the cylindrical tube body 130, the inner hole of the communication tube 11' may be the cylindrical hole and no tapered hole 114 is provided. When the through hole 134' is moved to be closed by the inner hole wall of the communication tube 11', the inverted tapered tube body 135' is abutted against a port of the inner hole of the communication tube 11', and the sealed off-state of the through hole 134' may also be achieved.

In the second embodiment, the connecting-driving mechanism includes an axial and circumferential coupling structure. The axial and circumferential coupling structure includes a third member arranged at an end of the outer tube 12' facing the sealing tube 13', and a fourth member arranged at a connecting end 131' of the sealing tube 13. The third member and the fourth member may be coupled with and decoupled from each other in the axial and circumferential directions. In FIG. 3, the third member is a first buckle 21', the fourth member is a second buckle 22'. The first buckle 21' and the second buckle 22' may be engaged with each other. In this embodiment, the first buckle 21' are two hooks arranged at the end of the outer tube 12' and extending in the circumferential direction, and the second buckle 22' are two hooks arranged at the end of the sealing tube 13' and extending in the circumferential direction. In some embodiments, the first buckle 21' and the second buckle 22' may be engaged to form a circle in the circumferential direction. In FIG. 3, the two hooks of the first buckle 21' at the end of the outer tube 12' are arranged opposite to each other in the circumferential direction, and the two hooks of the second buckle 22' at the end of the sealing tube 13' are also arranged opposite to each other in the circumferential direction, and correspond to the directions of the two hooks of the first buckle 21', respectively. During assembly, the first buckle 21' of the outer tube 12' can pass over the second buckle 22' of the sealing tube 13' in the axial direction, and the first buckle 21' of the outer tube 12' and the second buckle 22' of the sealing tube 13' move toward each other in the radial direction to be hooked and engaged with each other by means of concave-convex fit, so as to be connected with each other to form a circle in the circumferential direction. In some other embodiments, the two hooks of the first buckle 21' at the end of the outer tube 12' are arranged in the same direction in the circumferential direction (for example, both are arranged clockwise), and the two hooks of the second buckle 22' at the end of the sealing tube 13' are also arranged in the same direction in the circumferential direction, and correspond to the directions of the two hooks of the first buckle 21', respectively (for example, both are arranged counterclockwise). During assembly, the outer tube 12' and the sealing tube 13' are rotated oppositely in the circumferential direction, so that any hook of the first buckle 21' is hooked and engaged with (snapped to) a corresponding hook of the second buckle 22' by means of concave-convex fit. Then, the sealing tube 13' may be pulled in the axial direction by pulling the outer tube 12' in the axial direction, so that the sealing tube 13' is moved from the output end 112' of the communication tube 11' to the input end 111' of the communication tube 11', which allows the sealing tube 13' to block the communication tube 11'. When the sealing tube 13' is pulled in the axial direction to block the communication tube 11', the outer tube 12' and the sealing tube 13' may be rotated synchronously in the circumferential direction to increase the moving speed, so that the operation of blocking the communication tube 11' is more flexible and convenient for clinical application. Certainly, those of ordinary skill in the art can easily replace the first buckle 21' and the second buckle 22' with other structures, such as two locking teeth connected and matched with each other.

The sealing tube 13' and the outer tube 12' according to the second embodiment are snap-connected together and placed in the communication tube 11', and the self-on-off transmission assembly is arranged in the opening of the balloon body 1', thereby obtaining a balloon as shown in FIG. 5. The balloon body 1' is folded inside the puncture cannula, delivered to a predetermined part of the human body and released. In this case, the self-on-off transmission assembly is in the on-state, and the balloon body 1' is filled by injecting physiological saline through the outer tube 12'. Since the first sealing structure and the second sealing structure have a sealing effect, the filling material may not be seeped out between the outer tube 12' and the communication tube 11', and between the sealing tube 13' and the communication tube 11'. When the balloon body 1' is required to be sealed, the outer tube 12' and the sealing tube 13' are pulled in the axial direction, so that the outer tube 12' and the sealing tube 13' move from the output end 112' of the communication tube 11' to the input end 111' of the communication tube 11'. When the outer tube 12' is completely exposed to the input end 111', the outer tube 12' and the sealing tube 13' are rotated oppositely in the circumferential direction, or the outer tube 12' are directly moved in the radial direction. As a result, the circumferential coupling between the hooks of the first buckle 21' and the hooks of the second buckle 22' may be decoupled. The outer tube 12' is pulled in the axial direction, the outer tube 12' can be completely detached from the communication tube 11' in the axial direction, while the through hole 134' is placed in the tube body of the communication tube 11' and is blocked by the inner hole wall of the communication tube 11'. In this case, the self-on-off transmission assembly is in a sealed off-state, and the input end 111' and the output end 112' of the communication tube 11' are not in communication with each other, and the physiological saline in the balloon body 1' does not overflow the opening.

### Third Embodiment

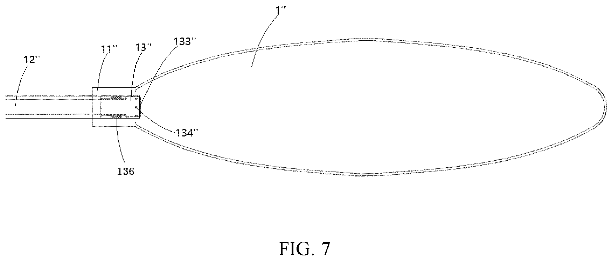

The third embodiment shown in FIG. 6 and FIG. 7 provides yet another self-on-off transmission assembly and a balloon. In this embodiment, the main structure is generally similar to those described in the first embodiment, and the main difference from those described in the first embodiment lies in structural forms of the first sealing structure, the second sealing structure and the connecting-driving mechanism. The first sealing structure between the outer peripheral surface of the outer tube 12" and the inner hole wall of the communication tube 11" is an elastic fit seal. For example, the outer tube 12" is inserted into the communication tube 11" from the input end 111", and the outer peripheral surface of the outer tube 12" is cooperated elastically with the inner hole wall of the communication tube 11", and thus the inner hole wall of the communication tube 11" is pressed against the outer peripheral surface of the outer tube 12" to achieve sealing.

In addition, in the third embodiment, the second sealing structure between the outer peripheral surface of the sealing tube 13" and the inner hole wall of the communication tube 11" includes a sealing ring 136 arranged on the outer periphery of the sealing tube 13". An outer peripheral surface of the sealing ring 136" is sealingly fitted with the inner hole wall of the communication tube 11". For example, the outer peripheral surface of the sealing ring 136 is in close contact with the inner hole wall of the communication tube 11", which may prevent the filling material from flowing to the input end 111" from the output end 112" of the communication tube 11" through a gap between the outer peripheral surface of the outer tube 12" and the inner hole walls of the communication tube 11".

The connecting-driving mechanism in the third embodiment includes an elastic coupling structure. The elastic coupling structure includes a plug-in cannula 135" arranged at a connecting end 131" of the sealing tube 13". The plug-in cannula 135" may be inserted into the inner hole of the end of the outer tube 12". When the outer tube 12" and the sealing tube 13" are placed in the communication tube 11", the end of the outer tube 12" is clamped between the communication tube 11" and the plug-in cannula 135". The inner hole wall of the communication tube 11" exerts a pressing force on the outer tube 12" in the radial direction, and the pressing force is transmitted to the plug-in cannula 135" in the radial direction. In this case, there is a large static friction force between the inner hole wall of the outer tube 12" and the outer peripheral surface of the plug-in cannula 135". When the outer tube 12" is pulled, the static friction force between the outer tube 12" and the plug-in cannula 135" allows the sealing tube 13" to be pulled synchronously in the axial direction, so that the sealing tube 13" is moved from the output end 112" of the communication tube 11" to the input end 111" of the communication tube 11", and thus the operation of blocking the communication tube 11" is simple and convenient for clinical application. In some embodiments, the communication tube 11", the sealing tube 13", and the outer tube 12" may be made of polyurethane, which has strong deformation ability. The outer tube 12" may be deformed when being subjected to the pressing force from the inner hole wall of the communication tube 11", which is beneficial for the pressing force to be transmitted to the plug-in cannula 135" in the radial direction, thereby increasing the static friction force between the inner hole wall of the outer tube 12" and the outer periphery surface of the plug-in cannula 135", causing the sealing tube 13" to be pulled more easily.

As can be seen from FIG. 6, the plug-in cannula 135" is connected to the sealing ring 136, and the sealing tube 13" further includes a transition ring 137 and an end ring 138. The transition ring 137 is connected between the sealing ring 136 and the end ring 138, and an diameter of the outer periphery the transition ring 137 is smaller than a hole diameter of the inner hole of the communication tube 11". The through hole 134" is arranged on a peripheral wall of the end ring 138, and an outer peripheral surface of the end ring 138 may be in elastic sealing fit with the inner hole wall of the communication tube 11".

The self-on-off transmission assembly shown in FIG. 6 is connected to the balloon body 1" to obtain a balloon as shown in FIG. 7. When the self-on-off transmission assembly is in the on-state, since the first sealing structure and the second sealing structure have a sealing effect, the physiological saline filled into the balloon body 1" does not seep out between the outer tube 12" and the communication tube 11", and between the sealing tube 13" and the communication tube 11". When the outer tube 12" and the sealing tube 13" are placed in the communication tube 11", the inner hole wall of the communication tube 11" exerts a pressing force on the outer tube 12" in the radial direction, so that a large static friction force is generated between the inner hole wall of the tube body of the outer tube 12" and the outer peripheral surface of the plug-in cannula 135". When the balloon body 1" is required to be sealed, the outer tube 12" is pulled in the axial direction, and the static friction force between the outer tube 12" and the plug-in cannula 135" allows the sealing tube 13" to be pulled synchronously in the axial direction, so that the sealing tube 13" is moved from the output end 112" to the input end 111" of the communication tube 11". When the outer tube 12" is completely exposed to the communication tube 11", the circumferential pressing force acting on the outer tube 12" by the inner hole wall of the communication tube 11"disappears, and the static friction force between the inner hole wall of the tube body of the outer tube 12" and the outer peripheral surface of the plug-in cannula 135" is reduced, so that the outer tube 12" may be easily separated from the sealing tube 13". In this case, the through hole 134" is placed in the tube body of the communication tube 11" and is blocked by the inner hole wall of the communication tube 11", and the output end 112" of the communication tube 11" is sealed by the sealing plate 133" of the sealing end 132". Thus, the self-on-off transmission assembly is in a sealed off-state, the input end 111" and the output end 112" of the communication tube 11" are not in communication with each other, and the physiological saline in the balloon body 1" may not overflow the opening.

In practical applications, the self-on-off transmission assembly described in the above embodiments can be directly used as a catheter connection product applied in a prosthesis of human joints, soft tissues, cavities or capsular bags. For example, the prosthesis includes a balloon, a catheter, and a catheter connector that connects the balloon to the catheter. A structure of the catheter connector is substantially the same as that of the above-mentioned self-on-off transmission assembly. When the prosthesis is implanted into the human body and the catheter connector is in an on-state, a filling medium may be injected into the balloon through the catheter. After filling, the catheter connector is operated to be in a sealed off-state. In this case, the balloon is in a sealed state, which may play the role of stable support and lubrication to ensure smooth and frictionless movement between bones, and help the organism restore the characteristics of the joint by replacing the damaged joint capsule or providing temporary support.

Any one of the communication tube, the outer tube, the sealing tube and the balloon body listed in the above embodiments is made of, but is not limited to be made of, polyamide, polyester, polyurethane, polyamide polyether block copolymer, polyethylene, polypropylene, polyimide, cross-linked polyethylene, cross-linked polyurethane, ionomer, polycaprolactone (PCL), polylactic acid (PLA), polylactic acid-glycolic acid copolymer (PLGA), lactide-caprolactone copolymer (PLCL), plastic starch materials, polyionic complexes, or copolymers or blends thereof. These materials involve biocompatible or biodegradable materials, which can be optimally matched according to the application environments. For example, if the implantable balloon is needed to replace organs in the organism, the biocompatible material is preferred; and if the balloon implantable is to provide an auxiliary function in the recovery period after the injury of the organism, the biodegradable material is preferred.

It can be understood that the connecting-driving mechanism, the first sealing structure, and the second sealing structure described in the above embodiments can be freely combined and matched in other embodiments as required. For example, the connecting-driving mechanism described in the second embodiment can also be directly used in the first embodiment, which does not affect the implementation of this application and is not limited herein.

## Claims

1. A medical self-on-off transmission assembly comprising:
a communication tube (11, 11', 11"), comprising an input end (111, 111', 111") and an output end (112, 112', 112");
an outer tube (12, 12', 12"), an end of the outer tube (12, 12', 12") being configured to be capable of being inserted into the communication tube (11, 11', 11") from the input end (111, 111', 111") and being moved out of the communication tube (11, 11', 11") from the input end (111, 111', 111"); and
a sealing tube (13, 13', 13"), configured to be capable of being placed into a tube body of the communication tube (11, 11', 11") and being moved in an axial direction with respect to the tube body of the communication tube (11, 11', 11"); the sealing tube (13, 13', 13") comprising a connecting end (131, 131', 131") and a sealing end (132, 132', 132"); the connecting end (131, 131', 131") being configured to be detachably connected to the end of the outer tube (12, 12', 12") through a connecting-driving mechanism; the sealing end (132, 132', 132") being provided with a communicating area;
wherein when the communicating area is exposed to the output end (112, 112', 112"), the self-on-off transmission assembly is in an on-state; when the communicating area is placed in the tube body of the communication tube (11, 11', 11"), the self-on-off transmission assembly is in a sealed off-state; and
wherein the outer tube (12, 12', 12") is configured to at least allow the self-on-off transmission assembly to be switchable from being in the on-state to being in the sealed off-state, by driving the sealing tube (13, 13', 13") to move along the communication tube (11, 11', 11") by the connecting-driving mechanism.

2. The medical self-on-off transmission assembly of claim 1, wherein the connecting-driving mechanism comprises a first member arranged at the end of the outer tube (12, 12', 12"), and a second member arranged at the connecting end (131, 131', 131") of the sealing tube (13, 13', 13"), and the first member and the second member are configured to be capable of being coupled with each other in a circumferential direction and decoupled from each other in the axial direction.

3. The medical self-on-off transmission assembly of claim 1, wherein the connecting-driving mechanism comprises a third member arranged at the end of the outer tube (12, 12', 12"), and a fourth member arranged at the connecting end (131, 131', 131") of the sealing tube (13, 13', 13"), and the third member and the fourth member are configured to be capable of being coupled with each other in the axial direction and in a circumferential direction, and being decoupled from each other in the axial direction and in the circumferential direction.

4. The medical self-on-off transmission assembly of claim 1, wherein the connecting-driving mechanism comprises a plug-in cannula (135") arranged at the connecting end (131, 131', 131") of the sealing tube (13, 13', 13"); the plug-in cannula (135") is configured to be capable of being inserted into an inner hole of the end of the outer tube (12, 12', 12"); when the end of the outer tube (12, 12', 12") is inserted into the communication tube (11, 11', 11"), the end of the outer tube (12, 12', 12") is clamped between the communication tube (11, 11', 11") and the plug-in cannula (135").

5. The medical self-on-off transmission assembly of claim 2, wherein the first member comprises a first arc-shaped cylinder (21), the second member comprises a second arc-shaped cylinder (22), and the first arc-shaped cylinder (21) and the second arc-shaped cylinder (22) are capable of being abutted against each other in the circumferential direction;
preferably, wherein an outer peripheral wall of the end of the outer tube (12, 12', 12") and an outer peripheral wall of the sealing tube (13, 13', 13") are both provided with outer threads; and an inner hole wall of the communication tube (11, 11', 11") is provided with inner threads matched with the outer threads.

6. The medical self-on-off transmission assembly of claim 3, wherein the third member is a first buckle (21'), the fourth member is a second buckle (22'), and the first buckle (21') and the second buckle (22') are capable of being engaged with each other;
preferably, wherein the first buckle (21') and the second buckle (22') are hooks extending in the circumferential direction.

7. The medical self-on-off transmission assembly of claim 3, wherein an inner hole of the communication tube (11, 11', 11") comprises a cylindrical hole (113) and a tapered hole (114) which are connected to each other, the tapered hole (114) is close to the output end (112, 112', 112"), the sealing tube (13, 13', 13") comprises a cylindrical tube body and an inverted tapered tube body which are connected to each other, the fourth member is arranged on the cylindrical tube body, the communicating area is arranged in the inverted tapered tube body, the cylindrical tube body is slidably matched with the cylindrical hole (113), and the inverted tapered tube body is adapted to be abutted against the tapered hole (114).

8. The medical self-on-off transmission assembly of claim 3, wherein the sealing tube (13, 13', 13") comprises a cylindrical tube body (130) and an inverted tapered tube body (135') which are connected to each other, the fourth member and the communicating area are arranged in the cylindrical tube body (130), the cylindrical tube body (130) is slidably matched with an inner hole of the communication tube (11, 11', 11"), and the inverted tapered tube body (135') is adapted to be abutted against an end of the communication tube (11, 11', 11").

9. The medical self-on-off transmission assembly of claim 4, wherein the sealing tube (13, 13', 13") comprises: a sealing ring (136) connected to the plug-in cannula (135"), an outer periphery surface of the sealing ring (136) being sealingly fitted with an inner hole wall of the communication tube (11, 11', 11"); an end ring (138), the communicating area being arranged on an periphery wall of the end ring (138), an outer periphery surface of the end ring (138) being capable of being sealingly fitted with the inner hole wall of the communication tube (11, 11', 11"); and a transition ring (137) connected between the sealing ring (136) and the end ring (138), a diameter of an outer periphery of the transition ring (137) being smaller than a diameter of an inner hole of the communication tube (11, 11', 11").

10. The medical self-on-off transmission assembly of claim 1, wherein a first sealing structure is arranged between an outer peripheral surface of the outer tube (12, 12', 12") and an inner hole wall of the communication tube (11, 11', 11"), and a second sealing structure is arranged between an outer peripheral surface of the sealing tube (13, 13', 13") and the inner hole wall of the communication tube (11, 11', 11");
preferably, wherein the first sealing structure is at least one of a threaded fitting seal, an interference fit seal, and an elastic fit seal.

11. The medical self-on-off transmission assembly of claim 10, wherein the second sealing structure comprises outer threads provided on the outer peripheral surface of the sealing tube (13, 13', 13"), and inner threads provided on the inner hole wall of the communication tube (11, 11', 11") and matched with the outer threads; or the second sealing structure is an inverted tapered tube body (135') arranged on the sealing tube (13, 13', 13") close to the sealing end (132, 132', 132"), a maximum circumferential dimension of the inverted tapered tube body (135') is greater than a circumferential dimension of an inner hole of the communication tube (11, 11', 11"); or the second sealing structure comprises a sealing ring (136) being sleeved on the sealing tube (13, 13', 13"), an outer peripheral surface of the sealing ring (136) is sealingly fitted with the inner hole wall of the communication tube (11, 11', 11").

12. The medical self-on-off transmission assembly of claim 1, wherein the communicating area is a through hole (134, 134', 134") or a slit located in a periphery wall of the sealing tube (13, 13', 13"), and the communicating area is capable of being blocked by an inner hole wall of the communication tube (11, 11', 11");
preferably, wherein a port of the sealing end (132, 132', 132") of the sealing tube (13, 13', 13") is sealed by a sealing plate (133, 133', 133").

13. A balloon comprising:
the medical self-on-off transmission assembly of any one of claims 1 to 12; and
a balloon body (1, 1', 1");
wherein the output end (112, 112', 112") of the communication tube (11, 11', 11") is connected to an opening of the balloon body (1, 1', 1").

14. The balloon of claim 13, wherein the communication tube (11, 11', 11"), the outer tube (12, 12', 12"), the sealing tube (13, 13', 13") and the balloon body (1, 1', 1") are made of polyamide, polyester, polyurethane, polyamide polyether block copolymer, polyethylene, polypropylene, polyimide, cross-linked polyethylene, cross-linked polyurethane, ionomer, polycaprolactone (PCL), polylactic acid (PLA), polylactic acid-glycolic acid copolymer (PLGA), lactide-caprolactone copolymer (PLCL), plastic starch materials, polyionic complexes, or copolymers or blends thereof.

15. A prosthesis for a shoulder joint, comprising a balloon, a catheter, and a catheter connector that connects the balloon to the catheter, wherein the catheter connector comprises:
a communication tube (11, 11', 11") comprising an input end (111, 111', 111") and an output end (112, 112', 112");
an outer tube (12, 12', 12"), an end of the outer tube (12, 12', 12") being configured to be capable of being inserted into the communication tube (11, 11', 11") from the input end (111, 111', 111") and being moved out of the communication tube (11, 11', 11") from the input end (111, 111', 111"); and
a sealing tube (13, 13', 13") configured to be capable of being placed into a tube body of the communication tube (11, 11', 11") and being moved in an axial direction with respect to the tube body of the communication tube (11, 11', 11"), the sealing tube (13, 13', 13") comprising a connecting end (131, 131', 131") and a sealing end (132, 132', 132"), the connecting end (131, 131', 131") being configured to be detachably connected to the end of the outer tube (12, 12', 12") through a connecting-driving mechanism, and the sealing end (132, 132', 132") being provided with a communicating area,
wherein when the communicating area is exposed to the output end (112, 112', 112"), the catheter connector is in an on-state; when the communicating area is placed in the tube body of the communication tube (11, 11', 11"), the catheter connector is in a sealed off-state; and
wherein the outer tube (12, 12', 12") is configured to at least allow the catheter connector to be switchable from being in the on-state to being in the sealed off-state, by driving the sealing tube (13, 13', 13") to move along the communication tube (11, 11', 11") by the connecting-driving mechanism.

## Patentansprüche

1. Medizinische selbst ein- und -ausschaltende Übertragungsanordnung, umfassend:
ein Kommunikationsrohr (11, 11', 11"), umfassend ein Eingangsende (111, 111', 111") und ein Ausgangsende (112, 112', 112");
ein Außenrohr (12, 12', 12"), wobei ein Ende des Außenrohrs (12, 12', 12") so konfiguriert ist, dass es vom Eingangsende (111, 111', 111") in das Kommunikationsrohr (11, 11', 11") eingeführt und vom Eingangsende (111, 111', 111") aus dem Kommunikationsrohr (11, 11', 11") herausbewegt werden kann; und
ein Dichtungsrohr (13, 13', 13"), das so konfiguriert ist, dass es in einen Rohrkörper des Kommunikationsrohrs (11, 11', 11") platziert und in der Axialrichtung relativ zum Rohrkörper des Kommunikationsrohrs (11, 11', 11") bewegt werden kann; wobei das Dichtungsrohr (13, 13', 13") ein Verbindungsende (131, 131', 131") und ein Dichtungsende (132, 132', 132") umfasst; wobei das Verbindungsende (131, 131', 131") so konfiguriert ist, dass es über einen Verbindungs-Antriebsmechanismus lösbar mit dem Ende des Außenrohrs (12, 12', 12") verbunden werden kann; wobei das Dichtungsende (132, 132', 132") mit einem Kommunikationsbereich bereitgestellt ist;
wobei, wenn der Kommunikationsbereich dem Ausgangsende (112, 112', 112") ausgesetzt ist, die selbst ein- und -ausschaltende Übertragungsanordnung sich in einem eingeschalteten Zustand befindet; wenn der Kommunikationsbereich in dem Rohrkörper des Kommunikationsrohrs (11, 11', 11") platziert ist, die selbst ein- und -ausschaltende Übertragungsanordnung sich in einem abgedichteten ausgeschalteten Zustand befindet; und
wobei das Außenrohr (12, 12', 12") so konfiguriert ist, dass es der selbst ein- und - ausschaltenden Übertragungsanordnung erlaubt, mindestens vom eingeschalteten Zustand in den abgedichteten ausgeschalteten Zustand umgeschaltet zu werden, indem das Dichtungsrohr (13, 13', 13") mittels des Verbindungs-Antriebsmechanismus so angetrieben wird, dass es sich entlang des Kommunikationsrohrs (11, 11', 11") bewegt.

2. Medizinische selbst ein- und -ausschaltende Übertragungsanordnung nach Anspruch 1, wobei der Verbindungs-Antriebsmechanismus ein erstes Element, das am Ende des Außenrohrs (12, 12', 12") angeordnet ist, und ein zweites Element umfasst, das am Verbindungsende (131, 131', 131") des Dichtungsrohrs (13, 13', 13") angeordnet ist, und das erste Element und das zweite Element so konfiguriert sind, dass sie in der Lage sind, in einer Umfangsrichtung miteinander gekoppelt und in der Axialrichtung voneinander entkoppelt zu werden.

3. Medizinische selbst ein- und -ausschaltende Übertragungsanordnung nach Anspruch 1, wobei der Verbindungs-Antriebsmechanismus ein drittes Element umfasst, das am Ende des Außenrohrs (12, 12', 12") angeordnet ist, und ein viertes Element, das am Verbindungsende (131, 131', 131") des Dichtungsrohrs (13, 13', 13") angeordnet ist, und das dritte Element und das vierte Element so konfiguriert sind, dass sie in der Lage sind, in der Axialrichtung und in einer Umfangsrichtung miteinander gekoppelt zu werden und in der Axialrichtung und in der Umfangsrichtung voneinander entkoppelt zu werden.

4. Medizinische selbst ein- und -ausschaltende Übertragungsanordnung nach Anspruch 1, wobei der Verbindungs-Antriebsmechanismus eine Steckkanüle (135") umfasst, die am Verbindungsende (131, 131', 131") des Dichtungsrohrs (13, 13', 13") angeordnet ist; wobei die Steckkanüle (135") so konfiguriert ist, dass sie in ein Innenloch des Endes des Außenrohrs (12, 12', 12") eingeführt werden kann; wobei, wenn das Ende des Außenrohrs (12, 12', 12") in das Kommunikationsrohr (11, 11', 11") eingeführt wird, das Ende des Außenrohrs (12, 12', 12") zwischen dem Kommunikationsrohr (11, 11', 11") und der Steckkanüle (135") eingeklemmt wird.

5. Medizinische selbst ein- und -ausschaltende Übertragungsanordnung nach Anspruch 2, wobei das erste Element einen ersten bogenförmigen Zylinder (21) umfasst, das zweite Element einen zweiten bogenförmigen Zylinder (22) umfasst, und der erste bogenförmige Zylinder (21) und der zweite bogenförmige Zylinder (22) in der Lage sind, in der Umfangsrichtung aneinander anzuliegen;
bevorzugt, wobei eine äußere Umfangswand des Endes des Außenrohrs (12, 12', 12") und eine äußere Umfangswand des Dichtungsrohrs (13, 13', 13") beide mit Außengewinden bereitgestellt sind; und eine innere Lochwand des Kommunikationsrohrs (11, 11', 11") mit Innengewinden bereitgestellt ist, die mit den Außengewinden übereinstimmen.

6. Medizinische selbst ein- und -ausschaltende Übertragungsanordnung nach Anspruch 3, wobei das dritte Element eine erste Schnalle (21') ist und das vierte Element eine zweite Schnalle (22') ist, und die erste Schnalle (21') und die zweite Schnalle (22') in der Lage sind, miteinander in Eingriff gebracht zu werden;
bevorzugt, wobei es sich bei der ersten Schnalle (21') und der zweiten Schnalle (22') um Haken handelt, die sich in der Umfangsrichtung erstrecken.

7. Medizinische selbst ein- und -ausschaltende Übertragungsanordnung nach Anspruch 3, wobei ein Innenloch des Kommunikationsrohrs (11, 11', 11") eine zylindrisches Loch (113) und ein konisches Loch (114) umfasst, die miteinander verbunden sind, das konische Loch (114) nahe am Ausgangsende (112, 112', 112") liegt, das Dichtungsrohr (13, 13', 13") einen zylindrischen Rohrkörper und einen umgekehrt konischen Rohrkörper umfasst, die miteinander verbunden sind, das vierte Element auf dem zylindrischen Rohrkörper angeordnet ist, der Kommunikationsbereich im umgekehrt konischen Rohrkörper angeordnet ist, der zylindrische Rohrkörper verschiebbar mit dem zylindrischen Loch (113) übereinstimmt und der umgekehrt konische Rohrkörper so angepasst ist, dass er an dem konischen Loch (114) anliegt.

8. Medizinische selbst ein- und -ausschaltende Übertragungsanordnung nach Anspruch 3, wobei das Dichtungsrohr (13, 13', 13") einen zylindrischen Rohrkörper (130) und einen umgekehrt konischen Rohrkörper (135') umfasst, die miteinander verbunden sind, das vierte Element und der Verbindungsbereich im zylindrischen Rohrkörper (130) angeordnet sind, der zylindrische Rohrkörper (130) verschiebbar mit einem Innenloch des Kommunikationsrohrs (11, 11', 11") übereinstimmt und der umgekehrt konische Rohrkörper (135') so angepasst ist, dass er an einem Ende des Kommunikationsrohrs (11, 11', 11") anliegt.

9. Medizinische selbst ein- und -ausschaltende Übertragungsanordnung nach Anspruch 4, wobei das Dichtungsrohr (13, 13', 13") umfasst: einen Dichtungsring (136), der mit der Steckkanüle (135") verbunden ist, wobei eine äußere Umfangsfläche des Dichtungsrings (136) dichtend mit der inneren Lochwand des Kommunikationsrohrs (11, 11', 11") abschließt; einen Endring (138), wobei der Kommunikationsbereich auf einer Umfangswand des Endrings (138) angeordnet ist, wobei eine äußere Umfangsfläche des Endrings (138) in der Lage ist, dichtend mit der inneren Lochwand des Kommunikationsrohrs (11, 11', 11") abschließbar zu sein; und einen Übergangsring (137), der zwischen dem Dichtungsring (136) und dem Endring (138) verbunden ist, wobei ein Durchmesser des äußeren Umfangs des Übergangsrings (137) kleiner ist als ein Durchmesser eines Innenloch des Kommunikationsrohrs (11, 11', 11").

10. Medizinische selbst ein- und -ausschaltende Übertragungsanordnung nach Anspruch 1, wobei eine erste Dichtungsstruktur zwischen einer äußeren Umfangsfläche des Außenrohrs (12, 12', 12") und einer inneren Lochwand des Kommunikationsrohrs (11, 11', 11") angeordnet ist und eine zweite Dichtungsstruktur zwischen einer äußeren Umfangsfläche des Dichtungsrohrs (13, 13', 13") und der inneren Lochwand des Kommunikationsrohrs (11, 11', 11") angeordnet ist;
bevorzugt, wobei die erste Dichtungsstruktur mindestens eine ist von einer Gewindedichtung, einer Presssitzdichtung oder einer elastischen Sitzdichtung.

11. Medizinische selbst ein- und -ausschaltende Übertragungsanordnung nach Anspruch 10, wobei die zweite Dichtungsstruktur Außengewinde umfasst, die an der äußeren Umfangsfläche des Dichtungsrohrs (13, 13', 13") bereitgestellt sind, und Innengewinde, die an der inneren Lochwand des Kommunikationsrohrs (11, 11', 11") bereitgestellt sind und mit den Außengewinden übereinstimmen; oder die zweite Dichtungsstruktur ein umgekehrt konischer Rohrkörper (135') ist, der auf dem Dichtungsrohr (13, 13', 13") nahe dem Dichtungsende (132, 132', 132") angeordnet ist, die maximale Umfangsabmessung des umgekehrt konischen Rohrkörpers (135') größer ist als die Umfangsabmessung eines Innenlochs des Kommunikationsrohrs (11, 11', 11"); oder die zweite Dichtungsstruktur einen Dichtungsring (136) umfasst, der auf das Dichtungsrohr (13, 13', 13") aufgeschoben ist, die äußere Umfangsfläche des Dichtungsrings (136) dichtend mit der inneren Lochwand des Kommunikationsrohrs (11, 11', 11") abschließt.

12. Medizinische selbst ein- und -ausschaltende Übertragungsanordnung nach Anspruch 1, wobei der Kommunikationsbereich ein Durchgangsloch (134, 134', 134") oder ein Schlitz ist, der sich in einer Umfangswand des Dichtungsrohrs (13, 13', 13") befindet, und der Kommunikationsbereich in der Lage ist, durch eine innere Lochwand des Kommunikationsrohrs (11, 11', 11") gesperrt zu werden;
bevorzugt, wobei ein Anschluss des Dichtungsendes (132, 132', 132") des Dichtungsrohrs (13, 13', 13") durch eine Dichtungsplatte (133, 133', 133") abgedichtet ist.

13. Ballon, umfassend:
die medizinische selbst ein- und -ausschaltende Übertragungsanordnung nach einem der Ansprüche 1 bis 12; und
einen Ballonkörper (1, 1', 1");
wobei das Ausgangsende (112, 112', 112") des Kommunikationsrohrs (11, 11', 11") mit einer Öffnung des Ballonkörpers (1, 1', 1") verbunden ist.

14. Ballon nach Anspruch 13, wobei das Kommunikationsrohr (11, 11', 11"), das Außenrohr (12, 12', 12"), das Dichtungsrohr (13, 13', 13") und der Ballonkörper (1, 1', 1") aus Polyamid, Polyester, Polyurethan, Polyamid-Polyether-Blockcopolymer, Polyethylen, Polypropylen, Polyimid, vernetztem Polyethylen, vernetztem Polyurethan, lonomer, Polycaprolacton (PCL), Polymilchsäure (PLA), Polymilchsäure-Glycolsäure-Copolymer (PLGA), Lactid-Caprolacton-Copolymer (PLCL), Kunststoffstärke, polyionischen Komplexen oder Copolymeren oder Mischungen davon bestehen.

15. Schultergelenkprothese, umfassend einen Ballon, einen Katheter und einen Katheterverbinder, der den Ballon mit dem Katheter verbindet, wobei der Katheterverbinder Folgendes umfasst:
ein Kommunikationsrohr (11, 11', 11"), das ein Eingangsende (111, 111', 111") und ein Ausgangsende (112, 112', 112") umfasst;
ein Außenrohr (12, 12', 12"), wobei ein Ende des Außenrohrs (12, 12', 12") so konfiguriert ist, dass es vom Eingangsende (111, 111', 111") in das Kommunikationsrohr (11, 11', 11") eingeführt und vom Eingangsende (111, 111', 111") aus dem Kommunikationsrohr (11, 11', 11") herausbewegt werden kann; und
ein Dichtungsrohr (13, 13', 13"), das so konfiguriert ist, dass es in einen Rohrkörper des Kommunikationsrohrs (11, 11', 11") platziert und in der Axialrichtung relativ zum Rohrkörper des Kommunikationsrohrs (11, 11', 11") bewegt werden kann; wobei das Dichtungsrohr (13, 13', 13") ein Verbindungsende (131, 131', 131") und ein Dichtungsende (132, 132', 132") umfasst; wobei das Verbindungsende (131, 131', 131") so konfiguriert ist, dass es über einen Verbindungs-Antriebsmechanismus lösbar mit dem Ende des Außenrohrs (12, 12', 12") verbunden werden kann, und das Dichtungsende (132, 132', 132") mit einem Kommunikationsbereich bereitgestellt ist,
wobei, wenn der Kommunikationsbereich dem Ausgangsende (112, 112', 112") ausgesetzt ist, der Katheterverbinder in einem eingeschalteten Zustand ist; wenn der Kommunikationsbereich in dem Rohrkörper des Kommunikationsrohrs (11, 11', 11") platziert ist, der Katheterverbinder in einem abgedichteten ausgeschalteten Zustand ist; und
wobei das Außenrohr (12, 12', 12") so konfiguriert ist, dass mindestens der Katheterverbinder vom eingeschalteten Zustand in den abgedichteten ausgeschalteten Zustand umgeschaltet werden kann, indem das Dichtungsrohr (13, 13', 13") mittels des Verbindungs-Antriebsmechanismus so angetrieben wird, dass es sich entlang des Kommunikationsrohrs (11, 11', 11") bewegt.

## Revendications

1. Ensemble de transmission médical à activation/désactivation automatique comprenant :
un tube de communication (11, 11', 11"), comprenant une extrémité d'entrée (111, 111', 111") et une extrémité de sortie (112, 112', 112") ;
un tube extérieur (12, 12', 12"), une extrémité du tube extérieur (12, 12', 12") étant configurée pour pouvoir être insérée dans le tube de communication (11, 11', 11") par l'extrémité d'entrée (111, 111', 111") et retirée du tube de communication (11, 11', 11") par l'extrémité d'entrée (111, 111', 111") ; et
un tube d'étanchéité (13, 13', 13"), configuré pour pouvoir être placé dans le corps tubulaire de communication (11, 11', 11") et déplacé dans une direction axiale par rapport au corps tubulaire du tube de communication (11, 11', 11") ; le tube d'étanchéité (13, 13', 13") comprenant une extrémité de raccordement (131, 131', 131") et une extrémité d'étanchéité (132, 132', 132") ; l'extrémité de raccordement (131, 131', 131") étant configurée pour être raccordée de manière détachable à l'extrémité du tube extérieur (12, 12', 12") par un mécanisme de raccordement-entraînement ; l'extrémité d'étanchéité (132, 132', 132") étant munie d'une zone de communication ;
dans lequel, lorsque la zone de communication est exposée à l'extrémité de sortie (112, 112', 112"), l'ensemble de transmission à activation/désactivation automatique est à l'état activé ; lorsque la zone de communication est placée dans le corps tubulaire du tube de communication (11, 11', 11"), l'ensemble de transmission à activation/désactivation automatique est à l'état désactivé étanche ; et
dans lequel le tube extérieur (12, 12', 12") est configuré pour permettre au moins à l'ensemble de transmission à activation/désactivation automatique de pouvoir passer de l'état activé à l'état désactivé étanche, en amenant le tube d'étanchéité (13, 13', 13") à se déplacer le long du tube de communication (11, 11', 11") par le mécanisme de raccordement-entraînement.

2. Ensemble de transmission médical à activation/désactivation automatique selon la revendication 1, dans lequel le mécanisme de raccordement-entraînement comprend un premier élément agencé à l'extrémité du tube extérieur (12, 12', 12") et un deuxième élément agencé à l'extrémité de raccordement (131, 131', 131") du tube d'étanchéité (13, 13', 13") et le premier élément et le deuxième élément sont configurés pour pouvoir être couplés l'un à l'autre dans une direction circonférentielle et découplés l'un de l'autre dans la direction axiale.

3. Ensemble de transmission médical à activation/désactivation automatique selon la revendication 1, dans lequel le mécanisme de raccordement-entraînement comprend un troisième élément agencé à l'extrémité du tube extérieur (12, 12', 12") et un quatrième élément agencé à l'extrémité de raccordement (131, 131', 131") du tube d'étanchéité (13, 13', 13") et le troisième élément et le quatrième élément sont configurés pour pouvoir être couplés l'un à l'autre dans la direction axiale et dans la direction circonférentielle et être découplés l'un de l'autre dans la direction axiale et dans la direction circonférentielle.

4. Ensemble de transmission médical à activation/désactivation automatique selon la revendication 1, dans lequel le mécanisme de raccordement-entraînement comprend une canule enfichable (135") agencée à l'extrémité de raccordement (131, 131', 131") du tube d'étanchéité (13, 13', 13") ; la canule enfichable (135") est configurée pour pouvoir être insérée dans un trou intérieur de l'extrémité du tube extérieur (12, 12', 12") ; lorsque l'extrémité du tube extérieur (12, 12', 12") est insérée dans le tube de communication (11, 11', 11"), l'extrémité du tube extérieur (12, 12', 12") est serrée entre le tube de communication (11, 11', 11") et la canule enfichable (135").

5. Ensemble de transmission médical à activation/désactivation automatique selon la revendication 2, dans lequel le premier élément comprend un premier cylindre arqué (21), le deuxième élément comprend un second cylindre arqué (22) et le premier cylindre arqué (21) et le second cylindre arqué (22) peuvent venir en butée l'un contre l'autre dans la direction circonférentielle ;
de préférence, dans lequel une paroi périphérique extérieure de l'extrémité du tube extérieur (12, 12', 12") et une paroi périphérique extérieure du tube d'étanchéité (13, 13', 13") sont toutes deux munies de filetages extérieurs ; et une paroi de trou intérieur du tube de communication (11, 11', 11") est munie de filetages intérieurs correspondant aux filetages extérieurs.

6. Ensemble de transmission médical à activation/désactivation automatique selon la revendication 3, dans lequel le troisième élément est une première boucle (21'), le quatrième élément est une seconde boucle (22'), et la première boucle (21') et la seconde boucle (22') peuvent venir en prise l'une avec l'autre ;
de préférence, dans lequel la première boucle (21') et la seconde boucle (22') sont des crochets s'étendant dans la direction circonférentielle.

7. Ensemble de transmission médical à activation/désactivation automatique selon la revendication 3, dans lequel un trou intérieur du tube de communication (11, 11', 11") comprend un trou cylindrique (113) et un trou conique (114) qui sont raccordés l'un à l'autre, le trou conique (114) est proche de l'extrémité de sortie (112, 112', 112"), le tube d'étanchéité (13, 13', 13") comprend un corps tubulaire cylindrique et un corps tubulaire conique inversé qui sont raccordés l'un à l'autre, le quatrième élément est agencé sur le corps tubulaire cylindrique, la zone de communication est agencée dans le corps tubulaire conique inversé, le corps tubulaire cylindrique est adapté de manière coulissante au trou cylindrique (113) et le corps tubulaire conique inversé est conçu pour venir en butée contre le trou conique (114).

8. Ensemble de transmission médical à activation/désactivation automatique selon la revendication 3, dans lequel le tube d'étanchéité (13, 13', 13") comprend un corps tubulaire cylindrique (130) et un corps tubulaire conique inversé (135') qui sont raccordés l'un à l'autre, le quatrième élément et la zone de communication sont agencés dans le corps tubulaire cylindrique (130), le corps tubulaire cylindrique (130) est adapté de manière coulissante à un trou intérieur du tube de communication (11, 11', 11") et le corps tubulaire conique inversé (135') est conçu pour venir en butée contre une extrémité du tube de communication (11, 11', 11").

9. Ensemble de transmission médical à activation/désactivation automatique selon la revendication 4, dans lequel le tube d'étanchéité (13, 13', 13") comprend : une bague d'étanchéité (136) reliée à la canule enfichable (135"), une surface périphérique extérieure de la bague d'étanchéité (136) étant ajustée de manière étanche avec une paroi de trou intérieur du tube de communication (11, 11', 11") ; une bague d'extrémité (138), la zone de communication étant agencée sur une paroi périphérique de la bague d'extrémité (138), une surface périphérique extérieure de la bague d'extrémité (138) pouvant être ajustée de manière étanche avec la paroi de trou intérieur du tube de communication (11, 11', 11") ; et une bague de transition (137) montée entre la bague d'étanchéité (136) et la bague d'extrémité (138), un diamètre d'une périphérie extérieure de la bague de transition (137) étant inférieur à un diamètre d'un trou intérieur du tube de communication (11, 11', 11").

10. Ensemble de transmission médical à activation/désactivation automatique selon la revendication 1, dans lequel une première structure d'étanchéité est agencée entre une surface périphérique extérieure du tube extérieur (12, 12', 12") et une paroi de trou intérieur du tube de communication (11, 11', 11") et une seconde structure d'étanchéité est agencée entre une surface périphérique extérieure du tube d'étanchéité (13, 13', 13") et la paroi de trou intérieur du tube de communication (11, 11', 11") ;
de préférence, dans lequel la première structure d'étanchéité est au moins l'une parmi un joint à ajustement par filetage, un joint à ajustement serré et un joint à ajustement élastique.

11. Ensemble de transmission médical à activation/désactivation automatique selon la revendication 10, dans lequel la seconde structure d'étanchéité comprend des filetages extérieurs disposés sur la surface périphérique extérieure du tube d'étanchéité (13, 13', 13") et des filetages intérieurs disposés sur la paroi de trou intérieur du tube de communication (11, 11', 11") et adaptés aux filetages extérieurs ; ou la seconde structure d'étanchéité est un corps tubulaire conique inversé (135') agencé sur le tube d'étanchéité (13, 13', 13") près de l'extrémité d'étanchéité (132, 132', 132"), une dimension circonférentielle maximale du corps tubulaire conique inversé (135') est supérieure à une dimension circonférentielle d'un trou intérieur du tube de communication (11, 11', 11") ; ou la seconde structure d'étanchéité comprend une bague d'étanchéité (136) étant emmanchée sur le tube d'étanchéité (13, 13', 13"), une surface périphérique extérieure de la bague d'étanchéité (136) étant ajustée de manière étanche avec la paroi de trou intérieur du tube de communication (11, 11', 11").

12. Ensemble de transmission médical à activation/désactivation automatique selon la revendication 1, dans lequel la zone de communication est un trou traversant (134, 134', 134") ou une fente située dans une paroi périphérique du tube d'étanchéité (13, 13', 13") et la zone de communication peut être bloquée par une paroi de trou intérieur du tube de communication (11, 11', 11") ;
de préférence, dans lequel un orifice de l'extrémité d'étanchéité (132, 132', 132") du tube d'étanchéité (13, 13', 13") est scellé par une plaque d'étanchéité (133, 133', 133").

13. Ballonnet comprenant :
l'ensemble de transmission médical à activation/désactivation automatique selon l'une quelconque des revendications 1 à 12 ; et
un corps de ballonnet (1, 1', 1") ;
dans lequel l'extrémité de sortie (112, 112', 112") du tube de communication (11, 11', 11") est raccordée à une ouverture du corps du ballonnet (1, 1', 1").

14. Ballonnet selon la revendication 13, dans lequel le tube de communication (11, 11', 11"), le tube extérieur (12, 12', 12"), le tube d'étanchéité (13, 13', 13") et le corps de ballonnet (1, 1', 1") sont en polyamide, polyester, polyuréthane, copolymère séquencé de polyamide et de polyéther, polyéthylène, polypropylène, polyimide, polyéthylène réticulé, polyuréthane réticulé, ionomère, polycaprolactone (PCL), acide polylactique (PLA), copolymère d'acide polylactique et d'acide glycolique (PLGA), copolymère de lactide et de caprolactone (PLCL), matériaux plastiques à base d'amidon, complexes polyioniques ou copolymères ou mélanges de ceux-ci.

15. Prothèse pour une articulation d'épaule, comprenant un ballonnet, un cathéter et un raccord de cathéter reliant le ballonnet au cathéter, dans laquelle le raccord de cathéter comprend :
un tube de communication (11, 11', 11") comprenant une extrémité d'entrée (111, 111', 111") et une extrémité de sortie (112, 112', 112") ;
un tube extérieur (12, 12', 12"), une extrémité du tube extérieur (12, 12', 12") étant configurée pour pouvoir être insérée dans le tube de communication (11, 11', 11") par l'extrémité d'entrée (111, 111', 111") et retirée du tube de communication (11, 11', 11") par l'extrémité d'entrée (111, 111', 111") ; et
un tube d'étanchéité (13, 13', 13") configuré pour pouvoir être placé dans un corps tubulaire du tube de communication (11, 11', 11") et être déplacé dans une direction axiale par rapport au corps tubulaire du tube de communication (11, 11', 11"), le tube d'étanchéité (13, 13', 13") comprenant une extrémité de raccordement (131, 131', 131") et une extrémité d'étanchéité (132, 132', 132"), l'extrémité de raccordement (131, 131', 131") étant configurée pour être raccordée de manière détachable à l'extrémité du tube extérieur (12, 12', 12") par un mécanisme de raccordement-entraînement et l'extrémité d'étanchéité (132, 132', 132") étant munie d'une zone de communication,
dans laquelle, lorsque la zone de communication est exposée à l'extrémité de sortie (112, 112', 112"), le raccord de cathéter est à l'état activé ; lorsque la zone de communication est placée dans le corps tubulaire du tube de communication (11, 11', 11"), le raccord de cathéter est à l'état désactivé étanche ; et
dans laquelle le tube extérieur (12, 12', 12") est configuré pour permettre au moins au raccord de cathéter de passer de l'état activé à l'état désactivé étanche, en amenant le tube d'étanchéité (13, 13', 13") à se déplacer le long du tube de communication (11, 11', 11") par le mécanisme de raccordement-entraînement.
